# EUROPEAN PATENT APPLICATION

(11) **EP 3 199 088 A1**
(43) Date of publication of application: **02.08.2017**
(21) Application number: 16811293.6
(22) Date of filing: 05.04.2016
(51) Int. Cl.: A61B 1/00, A61B 1/04

(54) **ENDOSCOPE**

(30) Priority: 16.06.2015 JP 2015121392
(71) Applicant: Olympus Corporation, Hachioji-shi Tokyo 192-8507 (JP)
(72) Inventor: FUJITANI Kiwamu, Tokyo 192-8507 (JP); HATANO Keisuke, (JP)
(74) Representative: Gunzelmann, Rainer
(86) International application number: PCT/JP2016/061080
(87) International publication number: WO 2016/203818

(57) **Abstract**

An object of the present invention is to provide an endoscope that can reliably protect incorporated components in a bending portion of an insertion portion of the endoscope without impairing ease of assembly, and in order to achieve the object, an endoscope according to the present invention includes: a bending tube 24 including ring-like members 25c pivotally joined via a pair of pivots 25a facing each other or a pair of pivots 25b facing each other, the pair of pivots 25a and the pair of pivots 25b being disposed in an up/down direction or a left/right direction relative to a center axis Ax, the bending tube 24 being bendable in an arbitrary direction; a pair of first pull wire guides 27 provided between adjacent pivots so as to project inward of a ring-like member; a forceps channel 13 disposed so as to be contactable with the pair of first pull wire guides; and a pair of second pull wire guides 26 positioned at respective positions, in the ring-like member, at which the pair of second pull wire guides 26 substantially faces the pair of first pull wire guides, the pair of second pull wire guides 26 being provided so as to project inward of the ring-like member and being disposed so that a distance D1 between distal end portions extending toward an inner circumferential side of the ring-like member is a distance that is smaller than an outer diameter D2 of the forceps channel.

## Description

### Technical Field

The present invention relates to an endoscope including a bendable bending portion on the distal end side of an insertion portion.

### Background Art

Conventionally, endoscopes including an insertion portion having an elongated tube shape have widely been used in, for example, the medical field and the industrial field. From among the endoscopes, the medical endoscopes used in the medical field are each configured so that the insertion portion can be inserted to the inside of a subject, for example, a body cavity of a living body to observe an organ or the like and as necessary, perform various treatment of the organ or the like using a treatment instrument (forceps) inserted in a treatment instrument insertion channel (forceps channel) included in the endoscope. Also, the industrial endoscopes used in the industrial filed are each configured so that the insertion portion can be inserted to the inside of an object, for example, an apparatus or a facility such as a jet engine or a plant piping to, e.g., observe or inspect a state of the object, for example, a state of, e.g., a crack or corrosion.

In this type of conventional endoscopes, various ingenuities are exercised in the elongated tube-shaped insertion portion having flexibility, in consideration of, e.g., ease of insertion when the insertion portion is inserted into a subject or object and/or convenience when the inside of a body cavity is observed. For example, conventional endoscopes including a bending portion in a predetermined area on the distal end side of an insertion portion, the bending portion being configured so as to be bendable in a desired, up/down/left/right, direction relative to a longitudinal direction of the insertion portion upon a user operating an operation member on the hand side to pull or loosen pull wires joined to the operation member, have widely been used.

In this case, as the operation member for bending the bending portion, in general, various types, for example, a bending operation member of what is called a joystick type in which, for example, a rod-like member is tilted, as well as an operation member using a dial-shaped rotary operation member (what is called, a bending knob, a bending lever or the like) have been proposed and in practical use.

In an endoscope including a bending portion that is bendable in a desired, up/down/left/right, direction as stated above, in particular, incorporated components in, particularly, the bending portion of the insertion portion are highly likely to interfere with one another upon bending of the bending portion, and thus, it is desirable to take some measures to prevent damage of incorporated components occurring due to, such interference between the incorporated components. Therefore, for example, the endoscopes disclosed in, e.g., Japanese Patent Application Laid-Open Publication No. 2012-61221 include a plurality of restriction members each provided on an inner wall of a cylindrical portion of each of joint rings (bending pieces) selected at intervals of a predetermined number of joint rings, each of the plurality of restriction members projecting inward of the cylindrical portion and restricting radial movement of incorporated components in a bending portion when a bending portion, e.g., bends.

However, in the above means disclosed in, e.g., Japanese Patent Application Laid-Open Publication No. 2012-61221, the plurality of restriction members are provided inside the joint rings (bending pieces), and thus, at the time of, e.g., assembling of the endoscope, the restriction members may interfere with e.g., an assembling tool, which may impair ease of assembly of the endoscope.

Also, as with, e.g., the technique disclosed in, e.g., the above publication, even if radial movement of incorporated components in a bending portion is restricted by a plurality of restriction members, it may be difficult to sufficiently protect the respective incorporated components. For example, if the distal end side is bent so as to rotate with a treatment instrument (forceps) inserted in a treatment instrument insertion channel (forceps channel), a part of the treatment instrument insertion channel (forceps channel), hardness of which is increased by the insertion of the treatment instrument (forceps), may, e.g., bulge between restriction members and press other incorporated components such as an image pickup cable against an inner wall of a joint ring and damage the other incorporated components.

The present invention has been made in view of the aforementioned points, and an object of the present invention is to provide an endoscope having a structure that can reliably protect incorporated components in a bending portion of an insertion portion of the endoscope without impairing ease of assembly.

### Disclosure of Invention

### Means for Solving the Problem

In order to achieve the object, an endoscope according to an aspect of the present invention includes: a bending tube including ring-like members pivotally joined via a pair of pivots facing each other, the pair of pivots being disposed in an up/down direction or a left/right direction relative to a center axis, the bending tube being bendable in an arbitrary direction; a pair of first pull wire guides each provided between the adjacent pivots so as to project inward of a ring-like member from among the ring-like members; a forceps channel disposed so as to be contactable with the pair of first pull wire guides; and a pair of second pull wire guides positioned at respective positions, in the ring-like member, at which the pair of second pull wire guides substantially faces the pair of first pull wire guides, the pair of second pull wire guides being provided so as to project inward of the ring-like member and being disposed so that a distance between distal end portions extending toward an inner circumferential side of the ring-like member is a distance that is smaller than an outer diameter of the forceps channel.

The present invention enables provision of an endoscope having a structure that can reliably protect incorporated components in a bending portion of an insertion portion of the endoscope without impairing ease of assembly.

### Brief Description of the Drawings

Fig. 1 is a front view illustrating an outer appearance of an endoscope according to an embodiment of the present invention;
Fig. 2 is a right side view of the endoscope in Fig. 1;
Fig. 3 is a top view of the endoscope in Fig. 1;
Fig. 4 is a cross-sectional view illustrating a major part of a distal end portion and a bending portion of the endoscope in Fig. 1;
Fig. 5 is a cross-sectional view along line [5]-[5] in Fig. 4;
Fig. 6 is a cross-sectional view along line [6]-[6] in Fig. 4;
Fig. 7 is a perspective cross-sectional view illustrating a bending piece in the endoscope in Fig. 1, the bending piece being broken into front and rear parts; and
Fig. 8 is a cross-sectional view illustrating a major part of a flexible tube portion of the endoscope in Fig. 1.

### Best Mode for Carrying Out the Invention

The present invention will be described below with reference to the embodiment illustrated in the drawings. Each of the drawings used for the below description is a schematic one, and in order to illustrate respective components in sizes that are large enough to be recognized in the drawings, the components may be illustrated so as to be different in, e.g., scale and dimensional relationship between members. Therefore, the present invention is not limited to the illustrated forms in terms of, e.g., the number of the components, shapes of the components, ratios in size between the components and relative positional relationships between the respective components indicated in each drawing.

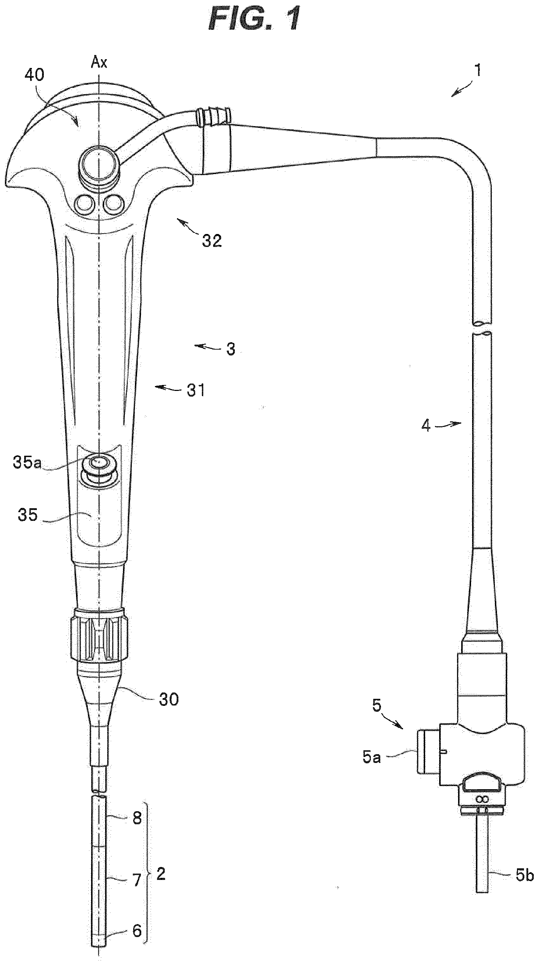

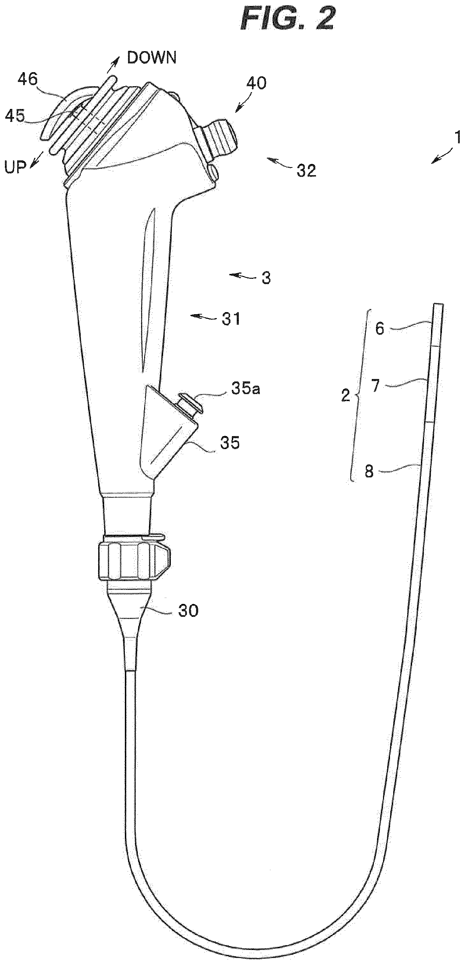

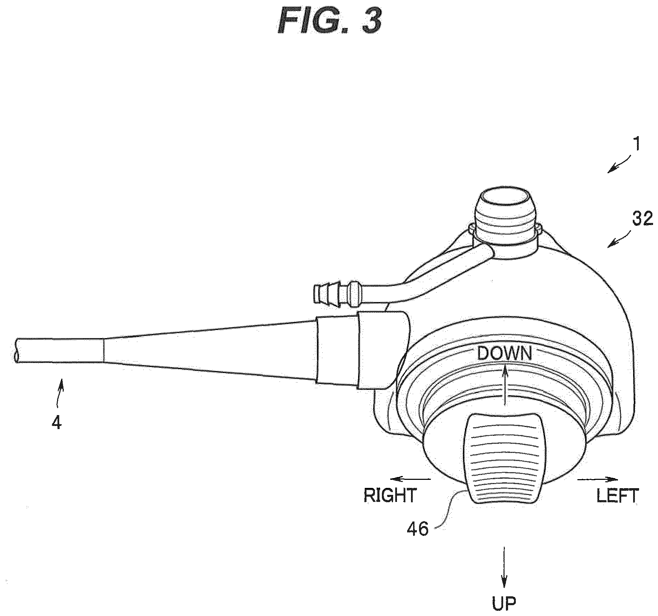

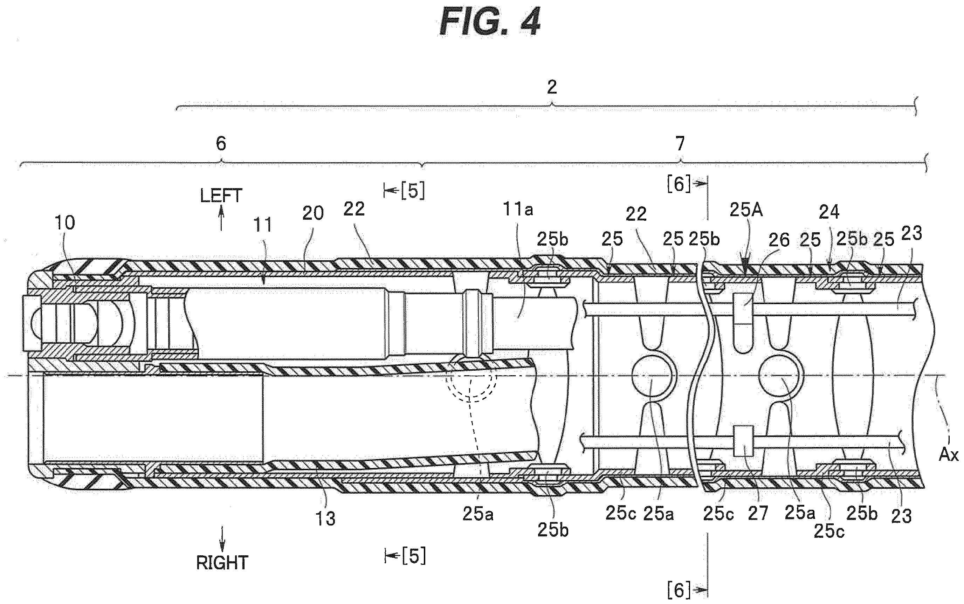

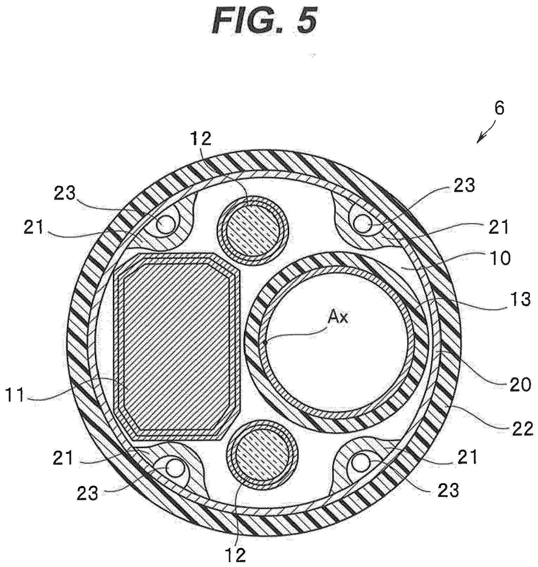

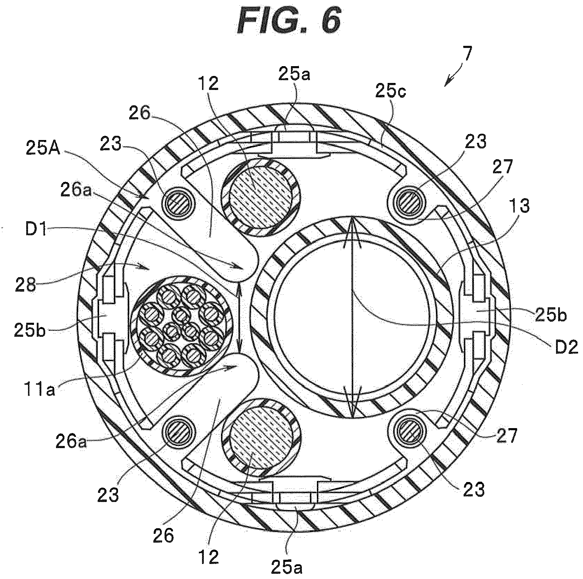

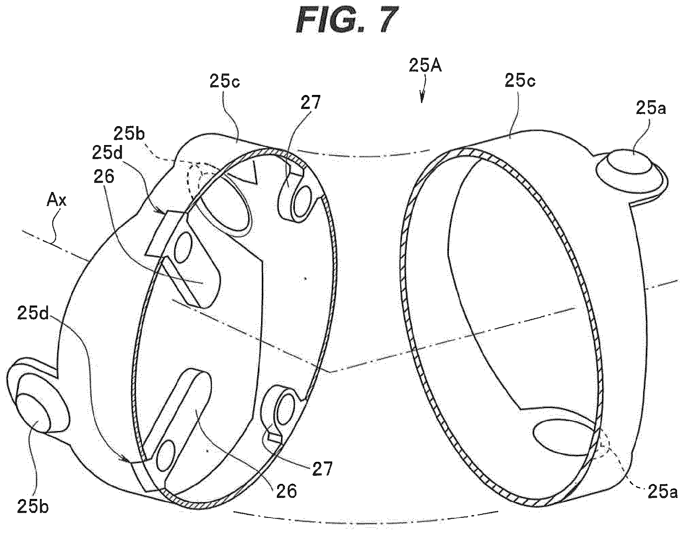

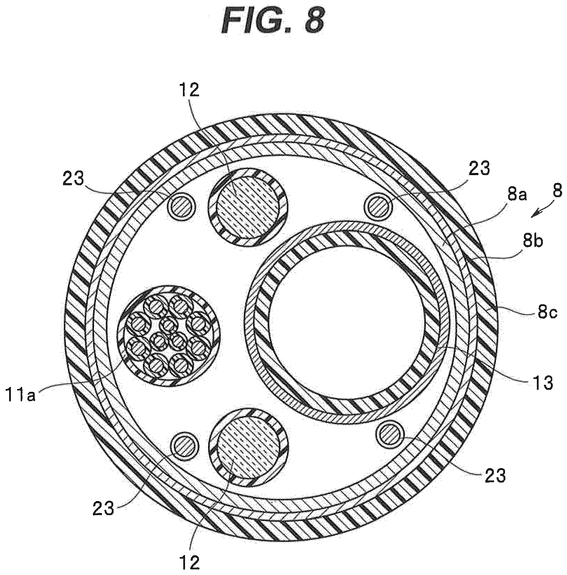

Fig. 1 is a front view illustrating an outer appearance of an endoscope according to an embodiment of the present invention. Fig. 2 is a right side view of the endoscope in Fig. 1. Fig. 3 is a top view of the endoscope in Fig. 1. Fig. 4 is a cross-sectional view illustrating a major part of a distal end portion and a bending portion of the endoscope in Fig. 1. Fig. 5 is a cross-sectional view along line [5]-[5] in Fig. 4. Fig. 6 is a cross-sectional view along line [6]-[6] in Fig. 4. Fig. 7 is a perspective cross-sectional view illustrating a bending piece broken into front and rear parts. Fig. 8 is a cross-sectional view illustrating a major part of a flexible tube portion.

First, an overall configuration of an endoscope 1 according to the present embodiment will be described below mainly with reference to Figs. 1 and 2. Here, the description will be provided taking a bronchoscope as an example of the endoscope 1 according the present embodiment.

As illustrated in Figs. 1 and 2, the endoscope 1 according to the present embodiment includes, e.g., an insertion portion 2 formed in an elongated tube shape, an operation portion 3 provided so as to be continuous with a proximal end of the insertion portion 2, a universal cord 4, which is an endoscope cable extending from the operation portion 3, and an endoscope connector 5 disposed at a distal end of the universal cord 4.

The insertion portion 2 includes a distal end portion 6, a bending portion 7 and a flexible tube portion 8 provided continuously in this order from the distal end side and is configured as a flexible tubular member as a whole.

Inside the distal end portion 6, as illustrated in Figs. 4 and 5, a distal end rigid portion 10 formed of a metal is provided, and in the distal end rigid portion 10, e.g., an image pickup unit 11 that incorporates an image pickup device such as CCD or CMOS, a plurality (for example, a pair) of light guides 12 and a treatment instrument insertion channel (also referred to as "forceps channel") 13 are held.

Also, inside the distal end portion 6, a most distal end bending piece 20 having a substantially cylindrical shape is fitted on the proximal end side of the distal end rigid portion 10, and an outer circumference of the most distal end bending piece 20 is covered by bending rubber 22. At each of four positions around an insertion axis (center axis of the insertion portion 2) Ax in an inner circumference of the most distal end bending piece 20, a wire fixing portion 21 is provided, and respective distal ends of four pull wires 23 inserted in the insertion portion 2 are fixed to the respective wire fixing portions 21.

Here, in order to efficiently arrange the respective constituent members without an increase in diameter of the distal end portion 6, the image pickup unit 11 and the treatment instrument insertion channel 13, which are large-size members, are disposed side by side inside the distal end rigid portion 10 and the most distal end bending piece 20 (see Figs. 4 and 5), and in spaces formed on the upper and lower sides as a result of such arrangement, the respective light guides 12 are disposed.

Also, in order to avoid interference between the image pickup unit 11 and the treatment instrument insertion channel 13, and each pull wire 23, each wire fixing portion 21 is provided at a position rotated around the insertion axis Ax by a predetermined angle relative to a top, bottom, left or right position of the distal end portion 6. In other words, for example, as illustrated in Fig. 5, the respective wire fixing portions 21 are provided at positions rotated to the left and the right, respectively, around the insertion axis Ax within a range of 15 to 70 degrees with reference to an up direction of the distal end portion 6 and positions rotated to the left and the right, respectively, around the insertion axis Ax within a range of 15 to 70 degrees with reference to a down direction of the distal end portion 6 in the most distal end bending piece 20.

The bending portion 7 includes a bending tube 24 configured to be actively bendable in any direction around the insertion axis Ax including up, down, left and right directions in response to an input provided via operation of the operation portion 3 by, e.g., a surgeon.

In an inner portion of the bending tube 24, an image pickup cable 11a for signal transmission (see Fig. 4), the image pickup cable 11a extending from the image pickup unit 11, the pair of light guides 12 and the treatment instrument insertion channel 13 are inserted as incorporated components, together with the respective pull wires 23 in an arrangement that is substantially similar to the arrangement inside the distal end portion 6. Furthermore, an outer circumference of the bending tube 24 is covered by the bending rubber 22 extending from the distal end portion 6 side.

The flexible tube portion 8 includes, for example, as illustrated in Fig. 8, a spiral sleeve 8a, a braid 8b covering an outer circumference of the spiral sleeve 8a and an outer covering 8c covering an outer circumference of the braid 8b. In an inner portion of the flexible tube portion 8, e.g., the image pickup cable 11a, the treatment instrument insertion channel 13 and the pair of light guide cable 12, which are mentioned above, are inserted. Also, the aforementioned four pull wires 23 are also inserted at respective predetermined positions.

Also, as illustrated in Figs. 1 and 2, the operation portion 3 includes a bend preventing portion 30 connected to the flexible tube portion 8 so as to cover a proximal end of the flexible tube portion 8, and an operation portion body 32 provided so as to be continuous with the bend preventing portion 30. Here, in the present embodiment, directions and the like around the insertion axis Ax in the operation portion 3 are defined with a state in which a user or the like grasps a grasping portion 31 as a reference, and more specifically, in the operation portion 3, front/rear and left/right directions (e.g., a front face, a back face and left and right side faces) are defined with a user or the like grasping the grasping portion 31 as a reference.

As illustrated in Fig. 1, in a front face on the distal end side of the grasping portion 31, a treatment instrument insertion portion 35 is provided. The treatment instrument insertion portion 35 includes a treatment instrument insertion opening 35a through which any of various types of treatment instruments (not illustrated) is inserted. In an inner portion of the operation portion 3, the treatment instrument insertion channel 13 communicates with the treatment instrument insertion opening 35a via a non-illustrated bifurcation member. Also, a forceps stopper (not illustrated), which is a cap member for occluding the treatment instrument insertion opening 35a, is attachable/detachable to the treatment instrument insertion portion 35.

On the front side of the operation portion body 32, an operation button group 40 for executing various functions of the endoscope 1 is disposed. On the other hand, on the back side of the operation portion body 32, a bending lever 45, which is a bending lever to be operated to bend the bending portion 7, is disposed. Furthermore, the universal cord 4 extends out from a side portion (for example, a left side portion) of the operation portion body 32.

Here, as illustrated in Figs. 2 and 3, the bending lever 45 in the present embodiment is configured by, for example, a joystick-type lever that is tiltable in any direction including the up, down, left and right directions. At a tip portion of the bending lever 45, a finger rest portion 46 on which, e.g., a thumb of a user to abut on is provided. Then, upon the bending lever 45 being operated so as to tilt through the finger rest portion 46, the respective pull wires 23 are properly pulled or loosened by a non-illustrated wire pulling mechanism disposed inside the operation portion body 32, enabling the bending portion 7 to bend in a desired bending direction.

Here, for example, as illustrated in Fig. 3, as tilting directions of the bending lever 45, for example, a left/right direction of a tilting operation is defined as a width, left/right, direction of the operation portion 3, which is a direction perpendicular to the insertion axis Ax, and an up/down direction is defined as a direction perpendicular to the width, left/right, direction. Then, the distal end side of the bending portion 7 can be bent so as to rotate by operating the bending lever 45 to tilt so as to draw a circle.

The universal cord 4 is a composite cable extending from the distal end portion 6 side to the operation portion 3 through the inside of the insertion portion 2, in which, e.g., various signal wires extending from the operation portion 3 are inserted, the light guides 12 from the light source apparatus (not illustrated) are inserted and an air/water feeding tube extending from an air/water feeding apparatus (not illustrated) is inserted.

The endoscope connector 5 includes an electric connector portion 5a to which a signal cable for connection with a video processor (not illustrated), which is an external device, at a side face portion, and a power supply connector portion 5b to which the light guides and an electric cable for connection with the light source apparatus, which is an external device, are connected.

Next, a detailed configuration of the bending portion 7 of the endoscope 1 according to the present embodiment will be described below.

As illustrated in Fig. 4, a bending tube 24 included in the bending portion 7 includes a plurality of bending pieces 25 serially joined along the insertion axis Ax direction. In other words, each bending piece 25 includes a bending piece body 25c, which is a ring-like member having a substantially cylindrical shape (ring shape), and the distal end sides and the proximal end sides of the bending piece bodies 25c, that is, the adjacent bending piece bodies 25c (ring-like members) are pivotally joined via a pair of pivots 25a facing each other or a pair of pivots 25b facing each other, which are alternately arranged in the up/down direction or the left/right direction relative to the insertion axis Ax (center axis) on the front side or the rear side, and the bending piece bodies 25c thus configure the bending tube 24 that is bendable in an arbitrary direction.

In such bending tube 24, as illustrated in Figs. 4, 6 and 7, in an inner portion of a particular bending piece 25A (provided with different reference numeral 25A for distinction from the other bending pieces 25) selected from the plurality of bending pieces 25, pull wire guides in which the four pull wires 23 are respectively inserted, the pull wire guides allowing movement in the insertion axis Ax of the respective pull wires 23 and restricting radial movement of the respective pull wires 23 inside the bending tube 24, are provided. Therefore, at sites close to respective proximal ends of the pull wire guides, insertion holes that allow the four pull wires 23 to be inserted respectively are provided so as to extend in a direction parallel to the insertion axis Ax. Here, the pull wire guides are provided at four sites corresponding to the four pull wires 23. From among the pull wire guides, two pull wire guides form a pair of first pull wire guides 27, and the other two pull wire guides form a pair of second pull wire guides 26.

The pair of first pull wire guides 27 is provided between pivots 25a and 25b adjacent to each other in a circumferential direction of the bending piece 25A so as to project inward of the bending piece body 25c (ring-like member). In this case, the pair of first pull wire guides 26 is disposed at respective positions which with the treatment instrument insertion channel 13 (forceps channel) can be in contact. In other words, the treatment instrument insertion channel 13 (forceps channel) is disposed so as to be contactable with the pair of first pull wire guides 26 inside the bending tube 24.

The pair of second pull wire guides 26 is fixedly provided at respective positions, in the bending piece body 25c (ring-like member), at which the pair of second pull wire guides 26 substantially faces the pair of first pull wire guides 27 with reference to the insertion axis Ax (center axis). In this case, the pair of second pull wire guides 26 is provided so as to project inward of the bending piece body 25c (ring-like member), and is disposed so that a distance D1 (see Fig. 6) between distal end portions of parts of the pair of second pull wire guides 26, the parts projecting toward the inner circumferential side of the bending piece body 25c (ring-like member), is smaller than an outer diameter D2 (see Fig. 6) of the treatment instrument insertion channel 13 (forceps channel).

With such configuration as above, the pair of second pull wire guides 26 receives the image pickup cable 11a in a space 28 formed between an inner circumferential face of the bending tube 24 and the pair of second pull wire guides 26 to restrict radial movement of the image pickup cable 11a inside the bending tube 24 and prevents entry of the treatment instrument insertion channel 13 (forceps channel) into the space 28 to suppress interference between incorporated components such as the image pickup cable 11a and the treatment instrument insertion channel 13 (forceps channel) inside the bending tube 24, and thus serves to protect relatively soft and vulnerable incorporated components such as the image pickup cable 11a from relatively rigid and durable incorporated components such as the treatment instrument insertion channel 13 (forceps channel).

The pair of second pull wire guides 26 is configured as bodies that are separate from the bending piece body 25c, and each second pull wire guide 26 is bonded to a predetermined site on an inner circumferential face of the bending piece body 25c via, for example, fixing means such as bonding or brazing. Consequently, each second pull wire guide 26 is fixed in such a manner that the second pull wire guide 26 is positioned at a predetermined position in the bending piece body 25c. Here, the means for fixing the second pull wire guide 26 to the bending piece body 25c is not limited to the aforementioned means such as brazing, and may be another means. For example, a structure in which each pull wire guide 26 is inserted to a hole portion 25d and a flange portion of the pull wire guide 26 abuts on a circumferential edge of the hole portion 25d may be provided to position and fix the pull wire guide 26 by biasing the pull wire guide 26 toward the bending piece body 25c via a pressing force of the bending rubber 22. As described above, the second pull wire guides 26 and the bending piece body 25c are configured in an integrated form.

With such configuration as above, when the bending tube 24 in the endoscope 1 according to the present embodiment is assembled, first, the adjacent bending piece bodies 25c (ring-like members) are pivotally joined at pivots 25a or 25b using means such as swaging processing. After the bending tube 24 being assembled as above, each of the pair of second pull wire guides 26 is inserted to a corresponding one of the plurality of hole portions 25d (see Fig. 7) provided in the bending piece body 25c (ring-like member) and is disposed in such a manner that the second pull wire guide 26 project inward of the bending piece body 25c (ring-like member). Then, with the position held, each second pull wire guide 26 is bonded and fixed to the bending piece body 25c via the aforementioned fixing means (for example, bonding or brazing).

Here, although the pair of second pull wire guides 26 is fixedly provided at the respective positions, in the bending piece body 25c (ring-like member), at which the pair of second pull wire guides 26 substantially faces the pair of first pull wire guides 27 with reference to the insertion axis Ax (center axis), the present invention is not limited to this form.

In other words, it is only necessary that the pair of second pull wire guides 26 be arranged at respective positions, in the inner circumferential face of the bending piece body 25c (ring-like member), at which the pair of second pull wire guides 26 substantially faces the pair of first pull wire guides 27. In this case, a direction of extension of the pair of second pull wire guides 26 to the inner circumferential side is not necessarily based on the insertion axis Ax (center axis), and it is only necessary that the pair of second pull wire guides 26 be disposed so as to project inward from the inner circumferential face of the bending piece body 25c (ring-like member).

Also, although in the above-described embodiment, a form in which the treatment instrument insertion channel 13 (forceps channel) from among the incorporated components in the insertion portion 2 of the endoscope 1 has a diameter that is larger than a diameter of the image pickup cable 11a for signal transmission, which extends from the image pickup unit 11, is indicated, the present invention is not limited to this form. For example, a form in which the treatment instrument insertion channel 13 (forceps channel) has a diameter that is smaller than the diameter of the image pickup cable 11a is possible. In the endoscope having such configuration, also, it is desirable that the distance between the distal end portions of the pair of second pull wire guides 26 be a distance that is smaller than the outer diameter of the treatment instrument insertion channel 13 (forceps channel).

As described above, according to the endoscope 1 of the above-described embodiment, in the bending tube 24 formed by joining the plurality of bending pieces 25, from among the pull wire guides for guiding movement of the four pull wires 23 arranged so as to be inserted in the inner portion of the bending tube 24 in the insertion axis Ax direction, respectively, each of the pair of second pull wire guides 26 that guide a part of (two) pull wires 23 is configured as is a body that is separate from the relevant bending piece body 25c, and the pair of second pull wire guides 26 is disposed so that the distance D1 between the distal end portions of the parts of the pair of second pull wire guides 26, the parts projecting toward the inner circumferential side of the bending piece body 25c (ring-like member), is a distance that is smaller than the outer diameter D2 of the treatment instrument insertion channel 13 (forceps channel).

As described above, as a result of the pair of second pull wire guides 26 and the bending piece bodies 25c being configured as separate bodies, at the time of work for assembling the bending tube 24, the second pull wire guides 26 can be positioned at respective predetermined positions and bonded after the bending piece bodies 25c are joined, which enables efficient assembly and thus can contribute to enhancement in ease of assembly and manufacturing cost reduction by assembling process simplification.

Also, as a result of the pair of second pull wire guides 26 being disposed so that the distance D1 between the distal ends of the pair of second pull wire guides 26 is a distance that is smaller than the outer diameter D2 of the treatment instrument insertion channel 13 (forceps channel), for example, a relatively soft and vulnerable incorporated component such as the image pickup cable 11 a is disposed in the space surrounded by the pair of second pull wire guides 26, enabling interference between the treatment instrument insertion channel 13 (forceps channel) and the image pickup cable 11a to be avoided, for example, even if the treatment instrument insertion channel 13 (forceps channel) greatly moves in the radial direction when the bending portion 7 is operated to bend so as to rotate, and thus enabling the image pickup cable 11a to be reliably protected. Therefore, the incorporated components in the bending portion 7 of the insertion portion 2 of the endoscope 1 can reliably be protected without impairing ease of assembly of the endoscope 1, which thus contributes to enhancement in durability of the incorporated components in the endoscope 1.

It should be understood that the present invention is not limited to the above-described embodiment and various alterations and applications are possible without departing from the spirit of the invention. Furthermore, the above-described embodiment includes various phases of the invention, and various aspects of the invention may be extracted by arbitrary combinations of the plurality of elements disclosed. For example, even where some elements are deleted from all the elements indicated in the above embodiment, a configuration with such elements deleted may be extracted as an aspect of the invention if such configuration can solve a problem to be solved by the invention and provide an effect of the invention. Furthermore, components in different embodiments may arbitrarily be combined. This invention is not restricted by any particular embodiment of the invention except as limited by the attached claims.

The present application is filed claiming priority from Japanese Patent Application No. 2015-121392 filed in Japan on June 16, 2015. The content disclosed in the above basic application is incorporated in the description, the claims and the drawings of the present application by reference.

### Industrial Applicability

The present invention is applicable not only to endoscope control apparatuses in the medical field, but also to endoscope control apparatuses in the industrial field.

## Claims

1. An endoscope comprising:
a bending tube including ring-like members pivotally joined via a pair of pivots facing each other, the pair of pivots being disposed in an up/down direction or a left/right direction relative to a center axis, the bending tube being bendable in an arbitrary direction;
a pair of first pull wire guides each provided between the adjacent pivots so as to project inward of a ring-like member from among the ring-like members;
a forceps channel disposed so as to be contactable with the pair of first pull wire guides; and
a pair of second pull wire guides positioned at respective positions, in the ring-like member, at which the pair of second pull wire guides substantially faces the pair of first pull wire guides, the pair of second pull wire guides being provided so as to project inward of the ring-like member and being disposed so that a distance between distal end portions extending toward an inner circumferential side of the ring-like member is a distance that is smaller than an outer diameter of the forceps channel.

2. The endoscope according to claim 1, wherein the image pickup cable is disposed in a space surrounded by the pair of second pull wire guides.

3. The endoscope according to claim 1, wherein the first and second pull wire guides are inserted from an outer circumferential side to the inner circumferential side of the ring-like member via a hole portion and positioned, the hole portion being provided at the ring-like member.
